# EUROPEAN PATENT SPECIFICATION

(11) **EP 3 845 131 B1**
(45) Date of publication and mention of the grant of the patent: **24.04.2024**
(21) Application number: 19853845.6
(22) Date of filing: 29.07.2019
(51) Int. Cl.: A61B 8/12, A61B 1/00, A61B 8/00

(54) **ULTRASOUND ENDOSCOPE BALLOON, ULTRASOUND ENDOSCOPE EQUIPPED WITH SAME, AND PRODUCTION METHOD THEREFOR**
ULTRASCHALLENDOSKOPBALLON, ULTRASCHALLENDOSKOP DAMIT UND HERSTELLUNGSVERFAHREN DAFÜR
BALLONNET D'ENDOSCOPE ULTRASONORE, ENDOSCOPE ULTRASONORE MUNI DE CE DERNIER, ET PROCÉDÉ DE FABRICATION CORRESPONDANT

(30) Priority: 27.08.2018 JP 2018158204
(43) Date of publication of application: 07.07.2021
(73) Proprietor: FUJIFILM Corporation, Tokyo 106-8620 (JP)
(72) Inventor: HAMADA, Kazuhiro, Ashigarakami-gun, Kanagawa 258-8577 (JP); NAKAI, Yoshihiro, Ashigarakami-gun, Kanagawa 258-8577 (JP)
(74) Representative: Hoffmann Eitle
(86) International application number: PCT/JP2019/029573
(87) International publication number: WO 2020/044905

(56) References cited:
- JP-A- H0 747 135
- JP-A- 2001 321 378
- JP-A- 2008 099 744
- JP-A- 2008 200 380
- JP-A- 2010 082 337
- JP-A- 2017 113 143
- JP-A- 2017 113 143
- JP-U- H 069 608
- US-A1- 2015 173 590
- US-A1- 2017 172 543

## Description

### BACKGROUND OF THE INVENTION

### 1. Field of the Invention

The present invention relates to a balloon for an ultrasonic endoscope, an ultrasonic endoscope including the balloon, and a method for producing the ultrasonic endoscope.

### 2. Description of the Related Art

Ultrasonic endoscopes are medical devices that have an ultrasonic transmission-reception section in a distal end portion of an endoscope insertion section and that perform ultrasonic diagnosis from the interior of the body. The ultrasonic transmission-reception section generates ultrasonic waves and receives echoes produced when the ultrasonic waves are applied to a subject. The received ultrasonic echoes are imaged, and, for example, diagnosis of the state of a surface and a deep part of the subject is performed.

In the transmission and reception of ultrasonic waves in the ultrasonic transmission-reception section, if air is present between the ultrasonic transmission-reception section and an observation site of the subject, the ultrasonic waves are attenuated and it becomes difficult to sufficiently obtain ultrasonic echoes necessary for image generation. To reduce this attenuation of ultrasonic waves, a balloon having elasticity is detachably attached so as to cover the ultrasonic transmission-reception section disposed in the insertion section of the ultrasonic endoscope. By filling a space formed between the endoscope insertion section and the balloon (balloon interior space) with a liquid substance such as water, the balloon is inflated to closely adhere to the observation site, and thus the attenuation of ultrasonic waves by air can be suppressed.

The attachment of the balloon to the endoscope insertion section is performed by fitting thick bead portions formed on both ends of the balloon into grooves provided in the endoscope insertion section. As for the material of the balloon, the use of various elastic members formed of, for example, natural rubber or a synthetic elastomer has been studied.

Patent Literature 1: Balloons for endoscopes are known from JP 2017 113143 A and US 2017/0172543 A1.

### SUMMARY OF THE INVENTION

As the material of the balloon, natural rubber is often used from the viewpoint of mechanical physical properties. However, natural rubber may cause a so-called latex allergy. In addition, for balloons formed of natural rubber, corn starch is used as an adhesion-preventing agent in order to reduce adhesion between balloons during shipping. However, this corn starch may also be a substance that causes an allergy.

In order to reduce the risk of developing these allergies, it is necessary to change to balloons produced by using synthetic elastomers as the materials. However, as a result of studies on balloons formed of synthetic elastomers, the inventors of the present invention have found that the balloons formed of synthetic elastomers tend to have poor mechanical physical properties compared with balloons formed of natural rubber. For example, it has been found that the balloons tend to easily break when the balloons are attached to endoscope insertion sections or when balloon interior spaces are filled with water.

Accordingly, an object of the present invention is to provide a balloon for an ultrasonic endoscope, the balloon being formed of a synthetic elastomer and unlikely to develop an allergy, capable of being firmly fixed to an endoscope insertion section, and unlikely to break in this fixed state. Another object of the present invention is to provide an ultrasonic endoscope including this balloon for an ultrasonic endoscope.

The inventors of the present invention have conducted extensive studies in view of the objects described above and found the following. Even in a balloon for an ultrasonic endoscope, the balloon being formed of a synthetic elastomer, when a width of a bead-portion attachment groove provided in an endoscope insertion section to fix the balloon and an outer diameter of a bead portion of the balloon to be attached to this groove have a specific relation, and an inner diameter of an opening of the balloon, the opening being surrounded by the bead potion, and an outer diameter of a part of the endoscope insertion section to which the balloon is to be attached have a specific relation, while the balloon is firmly fixed to the endoscope insertion section, mechanical physical properties of the balloon in this fixed state can also be enhanced.

Further studies have been conducted on the basis of these findings, and the present invention has been completed.

Specifically, the above objects of the present invention have been achieved by the following means.
[1] An ultrasonic endoscope having a balloon formed of a synthetic elastomer,
   in which the balloon is used such that a relation between a width W of a bead portion of the balloon and a width X of a bead-portion attachment groove provided in an ultrasonic endoscope insertion section to fix the balloon is 1.1 ≤ W/X ≤ 2.5, and
   a relation between an inner diameter Y of an opening of the balloon, the opening being surrounded by the bead portion, and an outer diameter Z of a part of the bead-portion attachment groove in the ultrasonic endoscope insertion section, the part being surrounded by a top of a wall surface of the bead-portion attachment groove, is 0.30 ≤ Y/Z ≤ 0.55.
[2] The ultrasonic endoscope according to [1], in which the synthetic elastomer is at least one of a styrene-based elastomer, an olefin-based elastomer, a vinyl chloride-based elastomer, a polyester-based elastomer, a polyurethane-based elastomer, a polyamide-based elastomer, a silicone-based elastomer, or a fluorine-based elastomer.
[3] The ultrasonic endoscope according to [1] or [2], in which the synthetic elastomer includes at least one olefin-based elastomer.
[4] The ultrasonic endoscope according to any one of [1] to [3], in which the synthetic elastomer includes isoprene rubber.
[5] The ultrasonic endoscope according to any one of [1] to [4], in which the balloon for an ultrasonic endoscope has a coating layer on at least an outer surface thereof.
[6] The ultrasonic endoscope according to [5], in which the coating layer is formed of a synthetic elastomer that is different from the synthetic elastomer forming the balloon for an ultrasonic endoscope.
[7] The ultrasonic endoscope according to [6], in which the synthetic elastomer used to form the coating layer is at least one of a styrene-based elastomer, an olefin-based elastomer, a vinyl chloride-based elastomer, a polyester-based elastomer, a polyurethane-based elastomer, a polyamide-based elastomer, a silicone-based elastomer, or a fluorine-based elastomer.
[8] The ultrasonic endoscope according to [6] or [7], in which the synthetic elastomer used to form the coating layer is at least one of a polyurethane-based elastomer, a silicone-based elastomer, or a fluorine-based elastomer.
[9] A method for producing an ultrasonic endoscope with a balloon attached to cover an ultrasonic transmission-reception section of an endoscope insertion section, the method including providing a balloon and an ultrasonic endoscope that satisfy relations (a) and (b) below; and attaching the balloon to an insertion section of the ultrasonic endoscope:
   (a) a relation between an outer diameter W of a bead portion of the balloon and a width X of a bead-portion attachment groove provided in the insertion section of the ultrasonic endoscope to fix the balloon is 1.1 ≤ W/X ≤ 2.5; and
   (b) a relation between an inner diameter Y of an opening of the balloon, the opening being surrounded by the bead portion, and an outer diameter Z of a part of the bead-portion attachment groove in the insertion section of the ultrasonic endoscope, the part being surrounded by a top of a wall surface of the bead-portion attachment groove, is 0.30 ≤ Y/Z ≤ 0.55.

In the description of the present invention, "to" is used in the meaning of a range that includes a numerical value described before "to" as a lower limit value and a numerical value described after "to" as an upper limit value.

The balloon for an ultrasonic endoscope according to the present invention is formed of a synthetic elastomer, which is unlikely to cause an allergy, can be firmly fixed to an endoscope insertion section, and is unlikely to break in this fixed state and thus has good mechanical physical properties. The ultrasonic endoscope according to the present invention includes the balloon for an ultrasonic endoscope according to the present invention so as to cover an ultrasonic transmission-reception section of an endoscope insertion section and enables, for example, diagnosis using the ultrasonic endoscope to be performed stably and efficiently.

### BRIEF DESCRIPTION OF THE DRAWINGS

Fig. 1 is an external view illustrating the configuration of an ultrasonic endoscope to which a balloon according to the present invention is to be attached;
Fig. 2 is a partial sectional view illustrating the configuration of a distal end portion of the ultrasonic endoscope illustrated in Fig. 1;
Fig. 3 is an external view illustrating an example of a balloon according to the present invention;
Fig. 4 is a sectional view of the balloon illustrated in Fig. 3; and
Fig. 5 is a partial sectional view illustrating a state in which a balloon is attached to the distal end portion of the ultrasonic endoscope illustrated in Fig. 2.

### DESCRIPTION OF THE PREFERRED EMBODIMENTS

Preferred embodiments of the present invention will be described below with reference to the drawings. The drawings illustrating the embodiments are each a schematic view provided for facilitating the understanding of the present invention. Regarding, for example, the sizes and the relative magnitude relations of members, the magnitudes may be changed for the convenience of description, and the drawings do not illustrate the actual relations as they are. In addition, the embodiments are not limited to the contours and the shapes illustrated in the drawings except for the matters specified in the present invention. Balloon for Ultrasonic Endoscope

A balloon for an ultrasonic endoscope according to the present invention (hereinafter, also simply referred to as a "balloon according to the present invention") is a member which is attached to cover an ultrasonic transmission-reception section disposed in an insertion section of an ultrasonic endoscope where a balloon interior space is filled with a liquid substance such as water in this state, and which suppresses attenuation of ultrasonic waves during the transmission and reception of the ultrasonic waves.

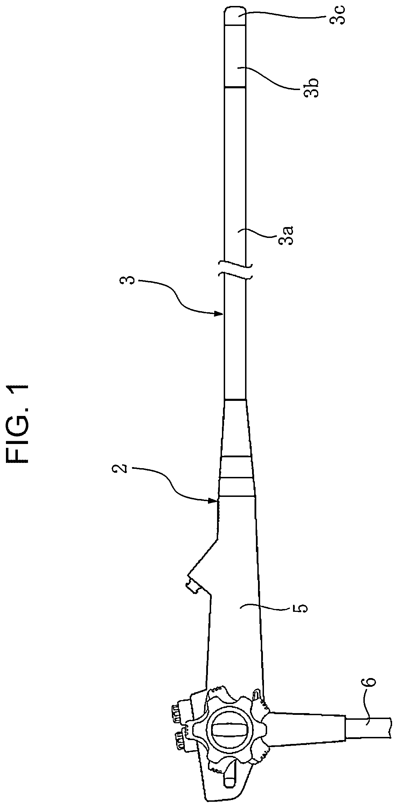

Fig. 1 illustrates an appearance (a basic configuration) of an example of an ultrasonic endoscope to which the balloon according to the present invention is to be attached. In the example illustrated in Fig. 1, an ultrasonic endoscope 2 includes an insertion section 3 to be inserted into a body cavity, a main-body operating section 5 connected to the proximal end portion of the insertion section 3, and a universal cord 6 connected to a processor device and a light source device. The insertion section 3 is constituted by a flexible tube 3a connected to the main-body operating section 5, an angle portion 3b connected to the flexible tube 3a, and a distal end portion 3c connected to the distal end of the angle portion 3b. The distal end portion 3c has an ultrasonic transducer (not illustrated) built therein for imaging the body cavity.

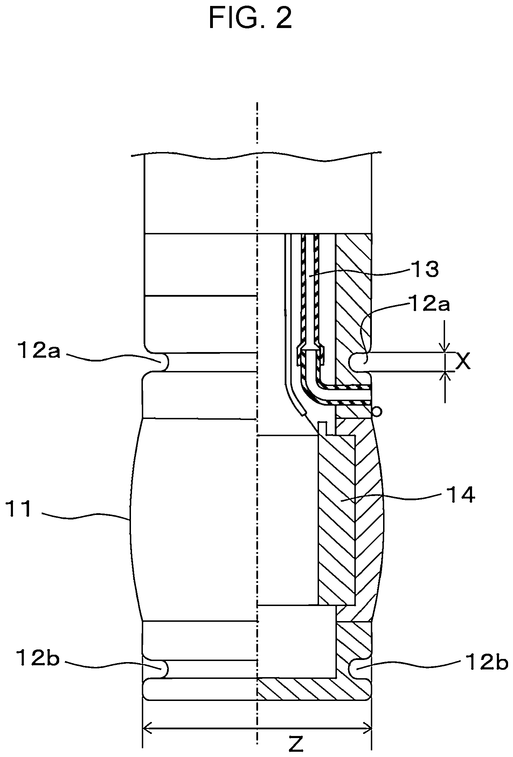

Fig. 2 illustrates a partial section of the configuration of the distal end portion 3c to which a balloon is to be attached, the distal end portion 3c being a part of the insertion section 3 of the ultrasonic endoscope illustrated in Fig. 1. This distal end portion 3c has an ultrasonic transmission-reception section 11 and is provided with bead-portion attachment grooves 12a and 12b to which a balloon is to be attached to cover the ultrasonic transmission-reception section. The distal end portion 3c has a pipe line 13 through which a liquid flows, the liquid filling a balloon interior space formed as a result of attachment of the balloon. Note that the cross sections of the distal end portion taken along the bead-portion attachment grooves 12a and 12b are usually each a surface perpendicular to the central axis of the distal end portion 3c.

An ultrasonic transducer 14 that performs conversion between electrical signals and ultrasonic waves is disposed in the ultrasonic transmission-reception section 11. The ultrasonic transducer 14 may have, for example, a radial scanning form in which a plurality of elements are arranged around the central axis of the columnar insertion section 3 in the circumferential direction.

A width of each of the bead-portion attachment grooves 12a and 12b illustrated in Fig. 2 (X in Fig. 2) is a "width X" specified in the present invention. In each of the bead-portion attachment grooves 12a and 12b illustrated in Fig. 2, an outer diameter of a part of the groove, the part being surrounded by a top of the wall surface of the groove, (Z in Fig. 2) is an "outer diameter Z" specified in the present invention. The "top of the wall surface of the groove" refers to a part where dropping starts from the surface of the distal end portion 3c toward the bottom of a bead-portion attachment groove in order to form the bead-portion attachment groove, as illustrated in Fig. 2.

The width X means a distance between two side faces of a bead-portion attachment groove in a part where the two side faces are formed to be parallel to each other. A bead portion of a balloon is fitted in at least the part where the two side faces of the bead-portion attachment groove are formed to be parallel to each other.

A cross section of the distal end portion 3c usually has a circular shape. Accordingly, a part of the bead-portion attachment groove, the part being surrounded by the top of the wall surface of the bead-portion attachment groove, (cross section of the distal end portion 3c taken along the top of the wall surface of the bead-portion attachment groove) also usually has a circular shape. In the case where the part of the bead-portion attachment groove, the part being surrounded by the top of the wall surface of the bead-portion attachment groove, does not have a circular shape, the "outer diameter Z" means an equivalent circle diameter. This "equivalent circle diameter" means a diameter of a perfect circle having the same area as the part of the bead-portion attachment groove, the part being surrounded by the top of the wall surface of the bead-portion attachment groove.

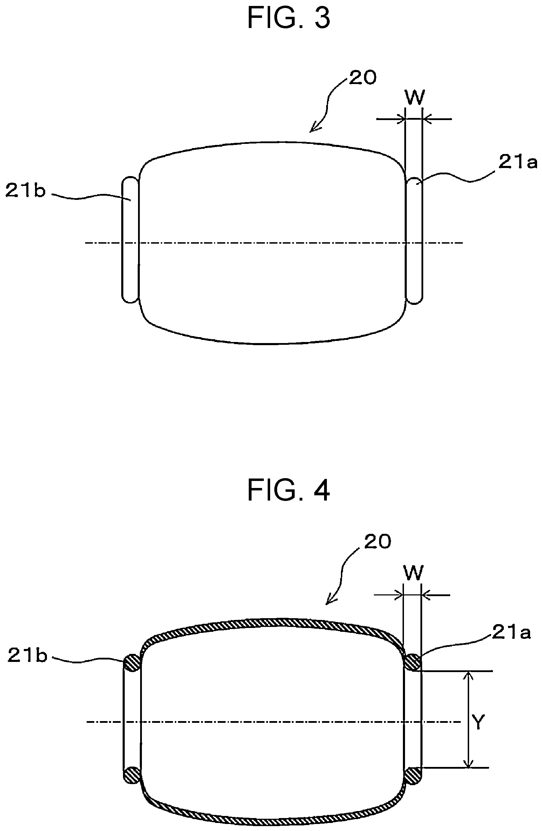

Fig. 3 is an external view illustrating an example of the balloon according to the present invention, and Fig. 4 is a sectional view of the balloon. A balloon 20 is formed to have a substantially cylindrical shape and attached to the distal end portion 3c of the insertion section 3 by inserting the distal end portion 3c therein. The balloon 20 has thick annular bead portions 21a and 21b on both ends thereof and is fixed to the distal end portion 3c by attaching the bead portions 21a and 21b to the bead-portion attachment grooves 12a and 12b (refer to Fig. 2), respectively, in a watertight manner.

Fig. 4 illustrates a "width W of a bead portion" and an "inner diameter Y of an opening of a balloon, the opening being surrounded by a bead portion" that are specified in the present invention. Here, the bead portion is inserted into a part where two side faces are formed to be parallel to each other in a bead-portion attachment groove provided in the endoscope insertion section. A maximum width of the bead portion in a direction perpendicular to these two side faces is the width W of the bead portion. The inner diameter Y means an inner diameter of an opening of the balloon, the opening being surrounded by the bead portion, in a state where the balloon is freestanding in a cylindrical shape (which means a state where the balloon is allowed to stand such that the central axis direction of the hole of the balloon corresponds to a lateral direction, hereinafter, the same). In the case where the opening of the balloon, the opening being surrounded by the bead portion, does not have a circular shape, an equivalent circle diameter is defined as the inner diameter Y. This "equivalent circle diameter" means a diameter of a perfect circle having the same area as the opening of the balloon, the opening being surrounded by the bead portion.

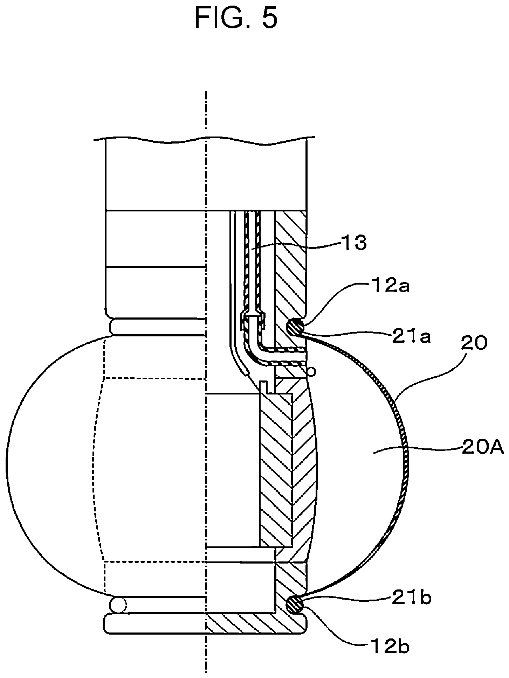

Fig. 5 is a partial sectional view illustrating a state in which a balloon 20 is attached to a distal end portion 3c of an ultrasonic endoscope. Bead portions 21a and 21b provided on both ends of the balloon 20 are respectively inserted into bead-portion attachment grooves 12a and 12b that are provided in the distal end portion 3c having an ultrasonic transmission-reception section and attached to the bead-portion attachment grooves 12a and 12b in a watertight manner to provide a state in which a balloon interior space 20A can be filled with a liquid such as water through a pipe line 13.

The balloon according to the present invention is formed of a synthetic elastomer. Specifically, the material for forming the balloon does not include natural rubber. Accordingly, the risk of developing the latex allergy caused by natural rubber can be reduced.

The balloon according to the present invention and an endoscope insertion section to which the balloon is to be attached satisfy the following relations (a1) and (b1).
(a1) The relation between the width W of a bead portion of the balloon and the width X of a bead-portion attachment groove provided in the endoscope insertion section to fix the balloon is 1.1 ≤ W/X ≤ 2.5.
(b1) The relation between the inner diameter Y of an opening of the balloon, the opening being surrounded by the bead portion, and the outer diameter Z of a part of the bead-portion attachment groove in the ultrasonic endoscope insertion section, the part being surrounded by a top of a wall surface of the bead-portion attachment groove, is 0.30 ≤ Y/Z ≤ 0.55.

The (a1) and (b1) above are relations between the balloon before the attachment to the endoscope insertion section (balloon in the unused state) and the ultrasonic endoscope insertion section (distal end portion) to which this balloon is to be attached.

In addition, the (a1) and (b1) above are relations between a bead portion at one end of the balloon and a bead-portion attachment groove of the distal end portion 3c in which the bead portion is to be inserted, and relations between a bead portion at the other end of the balloon and another bead-portion attachment groove of the distal end portion 3c in which the bead portion is to be inserted. For example, if the width of a bead portion at one end of the balloon is different from the width of a bead portion at the other end, or the size of an opening formed by a bead portion at one end of the balloon is different from the size of an opening formed by a bead portion at the other end, the above description means that the (a1) and (b1) above are satisfied in the relation between each of the bead portions and a bead-portion attachment groove corresponding to the bead portion. Alternatively, for example, if, in a bead portion at one end (or in a bead portion at the other end), the width W of the bead portion is not uniform and there is variation in the width W, the relation between the width W of all the parts of the bead portion at the one end (or the bead portion at the other end) and the width X of the attachment groove of this bead portion satisfies the (a1) above. Alternatively, for example, if there is variation in the width X of a bead-portion attachment groove of the distal end portion 3c, the relation between the groove width X in all the parts of the bead-portion attachment groove of the distal end portion 3c and the width W of the bead portion to be inserted into this groove satisfy the (a1) above. Alternatively, for example, if, in a bead portion at one end (or in a bead portion at the other end), the width W of the bead portion is not uniform, and in a bead-portion attachment groove in which this bead portion is to be inserted, there is also variation in the width X of the groove, the width W of all the parts of the bead portion at the one end (or the bead portion at the other end) and the groove width X in all the parts of the bead-portion attachment groove in which this bead portion is to be inserted satisfy the (a1) above.

In (a1) and (b1) above, the unit of the width W is the same as the unit of the width X (for example, each of the units is mm), and the unit of the inner diameter Y is also the same as the unit of the outer diameter Z (for example, each of the units is mm).

Satisfying the above relations enables the detachment and breakage of the balloon to be suppressed when the balloon interior space is filled with a liquid through the pipe line 13.

The relation between the width W of the bead portion and the width X of the bead-portion attachment groove preferably satisfies 1.6 ≤ W/X ≤ 2.4, more preferably satisfies 1.65 ≤ W/X ≤ 2.3, and still more preferably satisfies 1.7 ≤ W/X ≤ 2.3.

The dimension of the width W of the bead portion of the balloon is not particularly limited as long as the relation with the width X of the bead-portion attachment groove satisfies the above, and is preferably, for example, 0.5 to 3 mm, also preferably 1 to 2.5 mm, and more preferably 1.5 to 2.0 mm. As in the above case, if there is variation in the width of the bead portion of the balloon, the width of all the parts of the bead portion of the balloon preferably falls within the above preferred range.

The dimension of the inner diameter Y of the opening of the balloon, the opening being surrounded by the bead portion, is not particularly limited as long as the relation with the outer diameter Z of the part of the bead-portion attachment groove in the ultrasonic endoscope insertion section, the part being surrounded by the top of the wall surface of the bead-portion attachment groove, satisfies the above, and is preferably, for example, 1 to 8 mm, also preferably 2.5 to 6.5 mm, and more preferably 3.5 to 5.5 mm.

The length of the balloon according to the present invention is appropriately determined depending on the purpose. For example, in the state where the balloon is freestanding, the length from a bead portion at one end to a bead portion at the other end (the length of the balloon in the central axis direction) may be 10 to 100 mm, preferably 10 to 50 mm, and more preferably 20 to 40 mm. The length of the balloon in the central axis direction is preferably longer than the distance between the two bead-portion attachment grooves.

The relation of the (b1) above preferably satisfies 0.37 ≤ Y/Z ≤ 0.55, more preferably satisfies 0.38 ≤ Y/Z ≤ 0.55, and still more preferably satisfies 0.40 ≤ Y/Z ≤ 0.55.

Any synthetic polymer that exhibits rubber elasticity at room temperature (25°C) can be used as the synthetic elastomer for forming the balloon without particular limitation. For example, vulcanized rubber, rubber-like elastic materials, and thermoplastic elastomers can be widely used.

In particular, the synthetic elastomer used to form the balloon according to the present invention is preferably at least one of a styrene-based elastomer, an olefin-based elastomer, a vinyl chloride-based elastomer, a polyester-based elastomer, a polyurethane-based elastomer, a polyamide-based elastomer, a silicone-based elastomer, or a fluorine-based elastomer. Preferred examples of these elastomers will be described below; however, the present invention is not limited to these embodiments.

### Styrene-Based Elastomer

The styrene-based elastomer is an elastomer whose hard segment has a polystyrene structure. Examples of the styrene-based elastomer include styrene-butadiene block copolymers (SBR), hydrogenated styrene-butadiene block copolymers (SEB, styrene-ethylene/butylene block copolymers), styrene-butadiene-styrene block copolymers (SBS), hydrogenated styrene-butadiene-styrene block copolymers (SEBS, styrene-ethylene/butylene-styrene block copolymers), styrene-isoprene block copolymers (SIR), hydrogenated styrene-isoprene block copolymers (SEP, styrene-ethylene/propylene block copolymers), styrene-isoprene-styrene block copolymers (SIS), and hydrogenated styrene-isoprene-styrene block copolymers (SEPS, styrene-ethylene/propylene-styrene block copolymers). One or two or more of these can be used.

### Olefin-Based Elastomer

The olefin-based elastomer is a crosslinked or non-crosslinked elastomer having a polyolefin structure. Examples of the olefin-based elastomer include ethylene-propylene copolymers, ethylene-1-butene copolymers, ethylene-α-olefin copolymers, propylene-1-butene copolymers, propylene-α-olefin copolymers, 1-butene-α-olefin copolymers, propylene-1-butene-ethylene copolymers, propylene-α-olefin-ethylene copolymers, propylene-α-olefin-1-butene copolymers, 1-butene-α-olefin-ethylene copolymers, isoprene rubber (IR), cis-1,4-polybutadiene (BR), chloroprene rubber (CR), butyl rubber (IIR), elastomers in which ethylene-propylene rubber is dispersed in polypropylene, and elastomers in which ethylene-propylene-diene rubber is dispersed in polypropylene. One or two or more of these can be used.

### Vinyl Chloride-Based Elastomer

The vinyl chloride-based elastomer is an elastomer whose hard segment has a polyvinyl chloride structure. Examples of the vinyl chloride-based elastomer include polyvinyl chloride having a high degree of polymerization. Examples thereof further include an elastomer obtained by using a partially crosslinked polyvinyl chloride in which a crosslinked part functions as a hard segment and a linear part functions as a soft segment.

One or two or more of vinyl chloride-based elastomers can be used to form the balloon according to the present invention.

### Polyester-Based Elastomer

The polyester-based elastomer is an elastomer whose hard segment has a polyester structure. Examples thereof that can be used include block copolymers having a high-melting-point polyester segment (hard segment) and a low-melting-point polymer segment (soft segment) having a molecular weight of about 400 to 6,000 and described in, for example, JP1999-92636A (JP-H11-92636A). An example of the high-melting-point polyester segment is a polybutylene terephthalate (PBT). An example of the low-melting-point polymer segment is an amorphous polyether having a glass transition temperature of -70°C, e.g., polytetramethylene ether glycol (PTMG).

One or two or more of polyester-based elastomers can be used to form the balloon according to the present invention.

### Polyurethane-Based Elastomer

The polyurethane-based elastomer is an elastomer whose hard segment has a polyurethane structure. An example of the polyurethane-based elastomer is an elastomer including structural units of a hard segment formed from a low-molecular-weight glycol and a diisocyanate and a soft segment formed from a high-molecular-weight (long-chain) diol and a diisocyanate.

Examples of the high-molecular-weight (long-chain) diol include polypropylene glycol, polytetramethylene oxide, poly(1,4-butylene adipate), poly(ethylene-1,4-butylene adipate), polycaprolactone, poly(1,6-hexylene carbonate), and poly(1,6-hexylene/neopentylene adipate). The high-molecular-weight (long-chain) diol preferably has a number-average molecular weight of 500 or more and less than 10,000.

As the low-molecular-weight glycol, a short-chain diol such as ethylene glycol, propylene glycol, 1,4-butane diol, or bisphenol A can be used. The short-chain diol preferably has a number-average molecular weight of 48 or more and less than 500.

One or two or more of polyurethane-based elastomers can be used to form the balloon according to the present invention.

### Polyamide-Based Elastomer

The polyamide-based elastomer is an elastomer whose hard segment has a polyamide structure. Examples thereof include multiblock copolymers including a hard segment formed from a polyamide and a soft segment formed from a polyether or a polyester. Examples of the hard segment include polyamides 6, 66, 610, 11, and 12. Examples of the polyether in the soft segment include polyethylene glycol, diol poly(oxytetramethylene) glycol, and poly(oxypropylene) glycol. Examples of the polyester in the soft segment include poly(ethylene adipate)glycol and poly(butylene-1,4-adipate)glycol.

One or two or more of polyamide-based elastomers can be used to form the balloon according to the present invention.

### Silicone-Based Elastomer

The silicone-based elastomer is an elastomer having an organopolysiloxane structure. The silicone-based elastomer is an elastomer produced by introducing a crosslinked structure into an organopolysiloxane. For example, polydimethylsiloxane-based, polymethylphenylsiloxane-based, and polydiphenylsiloxane-based elastomers are known. Specific examples of a commercially available silicone-based elastomer include KE series (manufactured by Shin-Etsu Chemical Co., Ltd.) and SE series, CY series, and SH series (all of which are manufactured by Dow Corning Toray Silicone Co., Ltd.).

One or two or more of silicone-based elastomers can be used to form the balloon according to the present invention.

### Fluorine-Based Elastomer

The fluorine-based elastomer is an elastomer whose hard segment is formed from a fluororesin. Examples of the fluorine-based elastomer include tetrafluoroethylene-ethylene copolymers, tetrafluoroethylene-propylene copolymers, chlorotrifluoroethylene-ethylene copolymers, polyvinylidene fluoride, polyvinyl fluoride, vinylidene fluoride-hexafluoropropylene copolymers, tetrafluoroethylene-vinylidene fluoride-hexafluoropropylene copolymers, tetrafluoroethylene-vinylidene fluoride-perfluoroalkyl vinyl ether copolymers, tetrafluoroethylene-vinylidene fluoride-propylene copolymers, and tetrafluoroethylene-vinylidene fluoride-hexafluoropropylene copolymers.

One or two or more of fluorine-based elastomers can be used to form the balloon according to the present invention.

One or two or more of the synthetic elastomers described above can be used to form the balloon according to the present invention. The balloon according to the present invention preferably includes at least one olefin-based elastomer, and in particular, preferably includes isoprene rubber. The balloon according to the present invention is more preferably formed of at least one olefin-based elastomer and still more preferably formed of isoprene rubber.

The balloon according to the present invention preferably has a coating layer on at least an outer surface thereof. This coating layer can reduce adhesion between balloons during, for example, shipping and storage of the balloons. This coating layer is preferably formed of a synthetic elastomer. The above synthetic elastomers that can be used to form the balloon can be used as the synthetic elastomers that can be used to form the coating layer. In such a case, the synthetic elastomer used to form the balloon and the synthetic elastomer used to form the coating layer are preferably different types of synthetic elastomers. When the coating layer is formed of a synthetic elastomer, the synthetic elastomer used is preferably at least one of a polyurethane-based elastomer, a silicone-based elastomer, or a fluorine-based elastomer.

The balloon according to the present invention can be produced by a common method except that the width W of each bead portion of the balloon and the inner diameter Y of the opening of the balloon, the opening being surrounded by the bead portion, satisfy the specific relations described above together with the ultrasonic endoscope insertion section (distal end portion) to which the balloon is to be attached.

For example, a metallic mold having a desired size and shape is prepared, and the metallic mold is immersed in a dispersion liquid or solution of a polymer used for balloon formation to cause the polymer to adhere to the surface of the metallic mold. The adhering polymer is then dried, and the polymer adhering to the metallic mold is rolled up from an end such that the width of each bead portion and the inner diameter of the opening surrounded by the bead portion have desired dimensions. Subsequently, the polymer is removed from the metallic mold, optionally washed with, for example, water, and dried or performing a crosslinking reaction. Thus, a desired balloon can be produced. The width of the bead portion of the balloon and the inner diameter of the opening surrounded by the bead portion can be determined by observation with a microscope or the like.

When a thermoplastic elastomer is used as the material for forming the balloon, for example, extrusion molding or blow molding can also provide a balloon having a desired width of each bead portion and a desired inner diameter of the opening surrounded by the bead portion.

The coating layer of the balloon can be formed by a common method except that the balloon to be subjected to coating is the balloon according to the present invention. For example, the coating layer can be formed by immersing the balloon in a dispersion liquid, solution, or the like including a coating material or spraying a dispersion liquid, solution, or the like including a coating material on the balloon, and optionally subjecting the balloon to drying treatment or the like.

### Ultrasonic Endoscope

An ultrasonic endoscope according to the present invention is an ultrasonic endoscope including the balloon according to the present invention. The configuration of the balloon included in the ultrasonic endoscope according to the present invention and the configuration other than that of the balloon (that is, the configuration of the ultrasonic endoscope main body) are as described above.

The ultrasonic endoscope according to the present invention may be in a state where the balloon is attached. Alternatively, an ultrasonic endoscope before the attachment of the balloon and the balloon may be separated from each other. That is, an embodiment in which an ultrasonic endoscope before the attachment of a balloon and an unused balloon are separated from each other (a set of an ultrasonic endoscope and a balloon) is also encompassed by the ultrasonic endoscope according to the present invention.

The method for attaching the balloon to the insertion section (distal end portion) of the ultrasonic endoscope is not particularly limited. For example, the central axis of a tubular holding device and the central axis of the hole of the balloon are caused to substantially coincide with each other, and the balloon is placed in the hole of the holding device. While one end of a bead portion of the balloon is expanded by the hand, the expanded bead portion is folded back to the outer surface of the holding device so that the bead portion is fixed to a groove provided in the outer surface of the holding device, thus providing a state in which one end of the hole of the balloon is expanded to the size of the tube of the holding device. Subsequently, the insertion section (distal end portion) of the ultrasonic endoscope is inserted into the hole of the expanded balloon. The bead portion fixed to the groove in the outer surface of the holding device is moved to a bead-portion attachment groove (of the two bead-portion attachment grooves provided in the distal end portion, the bead-portion attachment groove provided on the angle portion side) of the endoscope insertion section (distal end portion). Thus, the one end of the bead portion is fixed to the endoscope insertion section. Subsequently, the holding device is removed, and water is supplied to fill the interior of the balloon with water. The other bead portion is expanded by the hand, and the bead portion expanded by the hand is fixed to the other bead-portion attachment groove of the endoscope insertion section while preventing air from entering the balloon interior space. Thus, the balloon can be attached to the insertion section (distal end portion) of the ultrasonic endoscope.

Specifically, the present invention provides a method for producing an ultrasonic endoscope with a balloon described below.

A method for producing an ultrasonic endoscope with a balloon attached to cover an ultrasonic transmission-reception section of an endoscope insertion section, the method including providing a balloon and an ultrasonic endoscope that satisfy relations (a) and (b) below, and attaching the balloon to the ultrasonic endoscope:
(a) a relation between an outer diameter W of a bead portion of the balloon and a width X of a groove provided in an ultrasonic endoscope insertion section to fix the balloon is 1.1 ≤ W/X ≤ 2.5 (preferably 1.6 ≤ W/X ≤ 2.4, and more preferably 1.65 ≤ W/X ≤ 2.3); and
(b) a relation between an inner diameter Y of an opening of the balloon, the opening being surrounded by the bead portion, and an outer diameter Z of a part of the bead-portion attachment groove in the ultrasonic endoscope insertion section, the part being surrounded by a top of a wall surface of the bead-portion attachment groove, is 0.30 ≤ Y/Z ≤ 0.55 (preferably 0.37 ≤ Y/Z ≤ 0.55, more preferably 0.38 ≤ Y/Z ≤ 0.55, and still more preferably 0.40 ≤ Y/Z ≤ 0.55).

Hereafter, the present invention will be described in more detail on the basis of Examples. However, the present invention is not limited by the Examples described below.

### EXAMPLES

### Examples 1 to 9 and Comparative examples 1 to 4

A balloon having the shape illustrated in Figs. 3 and 4 was produced.

A metallic mold having a shape corresponding to the shape of the balloon illustrated in Figs. 3 and 4 was immersed in a polyisoprene latex dispersion liquid (including a vulcanizing agent) to cause a polyisoprene latex to adhere to the surface of the metallic mold. Subsequently, the adhering polyisoprene latex was dried on the metallic mold. A balloon end was cut, and polyisoprene adhering to the metallic mold was rolled up from the end such that the width of a bead portion and the inner diameter of an opening surrounded by the bead portion were as described in Table 1, thus forming a bean portion. Two bead portions on both ends of the balloon had the same shape and the same size. The cross section of each of the bead portions had a circular shape as illustrated in Fig. 4. Subsequently, the polymer was removed from the metallic mold, washed with water, and vulcanized at 140°C. Thus, a balloon formed of isoprene rubber and shown in Table 1 was produced. The balloon produced as described above had, in the freestanding state, a length of 25 mm in the central axis direction of the balloon.

### Examples 10 to 19

The elastomer for forming a balloon was changed as shown in Table 1, and balloons shown in Table 1 were produced. The balloons of Examples 10 to 19 each had, in the freestanding state, a length of 25 mm in the central axis direction of the balloon.

Regarding Examples 11 and 12, the temperature of the elastomer for forming a balloon was increased to a temperature at which the elastomer had fluidity high enough for molding, and the elastomer was injection-molded in a metallic mold to produce a balloon.

Regarding Examples 18 and 19, the elastomer for forming a balloon was blow-molded to produce a balloon.

Regarding Examples 10, 13, 14, 15, 16, and 17, the elastomer for forming a balloon was dissolved in a solvent in which the elastomer is soluble, a metallic mold was immersed in the resulting solution and pulled up, and the solvent was then dried to produce a balloon.

In Test Examples described below, a scope having the shape illustrated in Fig. 2 was used as a distal end portion of an endoscope insertion section to which the balloon was to be attached. In this scope, the widths X of bead-portion attachment grooves are each 1 mm, the outer diameters Z of parts annularly surrounded by the tops of wall surfaces of the two bead-portion attachment grooves are each 10.7 mm, and the distance between the two bead-portion attachment grooves is 17.8 mm.

### Test Example 1

### Strength Test of Balloon Attached to Ultrasonic Endoscope Insertion Section

The balloon was attached to the above scope, and water was supplied through the pipe line to the balloon interior space at a flow rate of 100 mL/min. The time taken from the start of this water supply to breaking of the balloon was measured. A balloon having the same shape and size as the balloon used in the test was prepared by using natural rubber and used as a reference sample. The balloon strength was evaluated on the basis of evaluation criteria described below. At the time of the attachment of the balloon to the scope, breaking of the balloon had not yet occurred, and it was not until after the water supply in the Test Example that breaking occurred. In each of the cases, the breaking of the balloon did not occur in a bead portion but occurred in the vicinity of a bead portion.

### Evaluation Criteria of Balloon Strength

A: The time taken for the balloon to break is 1.8 times or more relative to that for the reference sample.
B: The time taken for the balloon to break is 1.6 times or more and less than 1.8 times relative to that for the reference sample.
C: The time taken for the balloon to break is 1.4 times or more and less than 1.6 times relative to that for the reference sample.
D: The time taken for the balloon to break is 1.2 times or more and less than 1.4 times relative to that for the reference sample.
E: The time taken for the balloon to break is 1.0 time or more and less than 1.2 times relative to that for the reference sample.
F: The time taken for the balloon to break is less than 1.0 time relative to that for the reference sample.

**Table 1**

| | Type of elastomer for forming balloon | Width W of bead portion [mm] | Inner diameter Y of opening surrounded by bead portion [mm] | [Width W]/[Width X] | [Inner diameter Y]/[Outer diameter Z] | Balloon strength |
|---|---|---|---|---|---|---|
| Example 1 | IR | 1.1 | 4.5 | 1.1 | 0.42 | D |
| Example 2 | IR | 1.5 | 4.5 | 1.5 | 0.42 | D |
| Example 3 | IR | 1.8 | 4.5 | 1.8 | 0.42 | A |
| Example 4 | IR | 2.2 | 4.5 | 2.2 | 0.42 | B |
| Example 5 | IR | 2.5 | 4.5 | 2.5 | 0.42 | C |
| Example 6 | IR | 1.8 | 3.2 | 1.8 | 0.30 | D |
| Example 7 | IR | 1.8 | 3.9 | 1.8 | 0.36 | C |
| Example 8 | IR | 1.8 | 5.1 | 1.8 | 0.48 | B |
| Example 9 | IR | 1.8 | 5.9 | 1.8 | 0.55 | B |
| Example 10 | BR | 1.8 | 4.5 | 1.8 | 0.42 | A |
| Example 11 | SEPS | 1.8 | 4.5 | 1.8 | 0.42 | A |
| Example 12 | SEBS | 1.8 | 4.5 | 1.8 | 0.42 | A |
| Example 13 | CR | 1.8 | 4.5 | 1.8 | 0.42 | A |
| Example 14 | IIR | 1.8 | 4.5 | 1.8 | 0.42 | A |
| Example 15 | PP-EPM | 1.8 | 4.5 | 1.8 | 0.42 | A |
| Example 16 | PP-EPDM | 1.8 | 4.5 | 1.8 | 0.42 | A |
| Example 17 | PUR | 1.8 | 4.5 | 1.8 | 0.42 | A |
| Example 18 | Si | 1.8 | 4.5 | 1.8 | 0.42 | A |
| Example 19 | F | 1.8 | 4.5 | 1.8 | 0.42 | A |
| Comparative Example 1 | IR | 1.0 | 4.5 | 1.0 | 0.42 | E |
| Comparative Example 2 | IR | 3.0 | 4.5 | 3.0 | 0.42 | E |
| Comparative Example 3 | IR | 1.8 | 3.0 | 1.8 | 0.28 | F |
| Comparative Example 4 | IR | 1.8 | 6.2 | 1.8 | 0.58 | Before breaking, balloon was detached from endoscope insertion section. |

Abbreviations shown in the above table are described below.
IR: isoprene rubber (trade name: CariflexTM IR-401, manufactured by Kraton Corporation)
BR: butadiene tuber (trade name: UBEPOL BR150B, manufactured by Ube Industries, Ltd.)
SEPS: styrene-ethylene/propylene-styrene block copolymer (trade name: SEPTON 4077, manufactured by Kuraray Co., Ltd.)
SEBS: styrene-ethylene/butylene-styrene block copolymer (trade name: SEPTON 8076, manufactured by Kuraray Co., Ltd.)
CR: chloroprene rubber (trade name: M-30, manufactured by Denka Company Limited)
IIR: butyl rubber (trade name: Butyl 365, manufactured by Japan Butyl Co., Ltd.)
PP-EPM: elastomer in which ethylene-propylene rubber is dispersed in polypropylene (trade name: THERMORUN Z103 N, manufactured by Mitsubishi Chemical Corporation)
PP-EPDM: elastomer in which ethylene-propylene-diene rubber is dispersed in polypropylene (trade name: THERMORUN QT70HG, manufactured by Mitsubishi Chemical Corporation)
PUR: polyurethane-based elastomer (trade name: MIRACTRAN E580, manufactured by Nippon Miractran Company Limited)
Si: silicone-based elastomer (trade name: KE-103, manufactured by Shin-Etsu Chemical Co., Ltd.), LSN201 (manufactured by FUJIKURA COMPOSITES Inc.)
F: fluorine-based elastomer (trade name: DAI-EL Thermoplastic T-530, manufactured by DAIKIN INDUSTRIES LTD.)

As shown in Table 1, when [Width W]/[Width X] was 1.0, the balloon could be fixed to the endoscope insertion section but easily broke in this fixed state (Comparative Example 1). Conversely, when [Width W]/[Width X] was larger than the values specified in the present invention, the balloon could be sufficiently fixed to the endoscope insertion section but also easily broke in this fixed state (Comparative Example 2). The reason why the balloon easily broke in the state of being fixed to the endoscope insertion section in Comparative Examples 1 and 2 is not clear.

When [Inner diameter Y]/[Outer diameter Z] was excessively small, the balloon easily broke (Comparative Example 3). Conversely, when [Inner diameter Y]/[Outer diameter Z] was excessively large, the balloon was detached from the endoscope insertion section before the balloon broke, and the balloon strength could not be evaluated (Comparative Example 4).

In contrast, the balloons satisfying the requirements of the present invention in terms of the relations with the endoscope insertion section each could be sufficiently fixed to the endoscope insertion section, and these balloons were resistant to the water pressure and unlikely to break (Examples 1 to 19).

### Examples 20 to 22

A balloon was produced as in Example 3. The balloon was immersed in a solution prepared by dissolving an elastomer for coating shown in the table below. The balloon was then dried. Thus, the outer surface of the balloon was coated with the elastomer different from the material for forming the balloon.

### Test Example 3

### Test for Reducing Adhesion between Balloons

With regard to the balloons of Examples 3 and 20 to 22, adhesiveness between balloons was tested by the method described below to examine a function of reducing adhesion, the function being achieved by the coating layer.

### Method for Testing Adhesiveness

Ten balloons that were identical to each other were randomly put in an aluminum Lami-Zip bag. The balloons were then allowed to stand for one night while a load of 3 kg was applied from above the aluminum Lami-Zip. Subsequently, the balloons were taken out from the aluminum Lami-Zip, and the occurrence or nonoccurrence of adhesion between the balloons was examined by visual observation. Specifically, in the case where one of the balloons was picked up, when none of balloons that had been in contact with the one balloon adhered to the one balloon or were picked up together with the one balloon, it was evaluated that adhesion did "not occur". When balloons that had been in contact with the one balloon were also picked up together with the one balloon, it was evaluated that adhesion "occurred".

The table below shows the results. The abbreviations in Table 2 have the same meaning as those in Table 1.

**Table 2**

| | Type of elastomer for forming balloon | Type of elastomer for forming coating layer | Occurrence or nonoccurrence of adhesion between balloons |
|---|---|---|---|
| Example 3 | IR | None | Occur |
| Example 20 | IR | Si | Not occur |
| Example 21 | IR | F | Not occur |
| Example 22 | IR | PUR | Not occur |

The abbreviations are the same as those in Table 1.

As shown in Table 2, adhesion between balloons can be prevented by providing a coating layer on a balloon formed of a synthetic elastomer. Accordingly, balloons that are easily handled even during storage and distribution can be provided by subjecting the balloons to coating treatment.

The present invention has been described together with embodiments thereof. However, we do not intend to limit our invention in any of the details of the description unless otherwise specified. We believe that the invention should be broadly construed without departing from the scope of the invention as defined by the appended claims

### Reference Signs List

2 ultrasonic endoscope
3 insertion section
   3a flexible tube
   3b angle portion
   3c distal end portion
5 main-body operating section
6 universal cord
11 ultrasonic transmission-reception section 12a, 12b bead-portion attachment groove X width of bead-portion attachment groove 13 pipe line
14 ultrasonic transducer
Z outer diameter of part of bead-portion attachment groove, the part being surrounded by top of wall surface of the bead-portion attachment groove
20 balloon
20A balloon interior space
21a, 21b bead portion
W width of bead portion of balloon
Y inner diameter of opening of balloon, the opening being surrounded by bead portion

## Claims

1. An ultrasonic endoscope (2) comprising a balloon (20), the balloon (20) comprising a synthetic elastomer,
**characterized in that** a relation between a width W of a bead portion (21a, 21b) of the balloon (20) and a width X of a bead-portion attachment groove (12a, 12b) provided in the ultrasonic endoscope (2) insertion section (3) to fix the balloon (20) is 1.1 ≤ W/X ≤ 2.5, and **in that**
a relation between an inner diameter Y of an opening of the balloon (20), the opening being surrounded by the bead portion (21a, 21b), and an outer diameter Z of a part of the bead-portion attachment groove (12a, 12b) in the ultrasonic endoscope (2) insertion section (3), the part being surrounded by a top of a wall surface of the bead-portion attachment groove (12a, 12b), is 0.30 ≤ Y/Z ≤ 0.55.

2. The ultrasonic endoscope (2) according to claim 1, wherein the synthetic elastomer is at least one of a styrene-based elastomer, an olefin-based elastomer, a vinyl chloride-based elastomer, a polyester-based elastomer, a polyurethane-based elastomer, a polyamide-based elastomer, a silicone-based elastomer, or a fluorine-based elastomer.

3. The ultrasonic endoscope (2) according to claim 1 or 2, wherein the synthetic elastomer includes at least one olefin-based elastomer.

4. The ultrasonic endoscope (2) according to any one of claims 1 to 3, wherein the synthetic elastomer includes isoprene rubber.

5. The ultrasonic endoscope (2) according to any one of claims 1 to 4, wherein the balloon (20) has a coating layer on at least an outer surface thereof.

6. The ultrasonic endoscope (2) according to claim 5, wherein the coating layer is formed of a synthetic elastomer that is different from the synthetic elastomer forming the balloon (20).

7. The ultrasonic endoscope (2) according to claim 6, wherein the synthetic elastomer used to form the coating layer is at least one of a styrene-based elastomer, an olefin-based elastomer, a vinyl chloride-based elastomer, a polyester-based elastomer, a polyurethane-based elastomer, a polyamide-based elastomer, a silicone-based elastomer, or a fluorine-based elastomer.

8. The ultrasonic endoscope (2) according to claim 6 or 7, wherein the synthetic elastomer used to form the coating layer is at least one of a polyurethane-based elastomer, a silicone-based elastomer, or a fluorine-based elastomer.

9. A method for producing an ultrasonic endoscope (2) with a balloon (20) attached to cover an ultrasonic transmission-reception section of an endoscope (2) insertion section (3), the method comprising:
providing a balloon (20) and an ultrasonic endoscope (2) that satisfy relations (a) and (b) below; and
attaching the balloon (20) to an insertion section (3) of the ultrasonic endoscope (2):
(a) a relation between an outer diameter W of a bead portion (21a, 21b) of the balloon (20) and a width X of a bead-portion attachment groove (12a, 12b) provided in the insertion section (3) of the ultrasonic endoscope (2) to fix the balloon (20) is 1.1 ≤ W/X ≤ 2.5; and
(b) a relation between an inner diameter Y of an opening of the balloon (20), the opening being surrounded by the bead portion (21a, 21b), and an outer diameter Z of a part of the bead-portion attachment groove (12a, 12b) in the insertion section (3) of the ultrasonic endoscope (2), the part being surrounded by a top of a wall surface of the bead-portion attachment groove (12a, 12b), is 0.30 ≤ Y/Z ≤ 0.55.

## Patentansprüche

1. Ultraschallendoskop (2), umfassend einen Ballon (20), wobei der Ballon (20) ein synthetisches Elastomer umfasst,
**dadurch gekennzeichnet, dass** ein Verhältnis zwischen einer Breite W eines Wulstabschnitts (21a, 21b) des Ballons (20) und einer Breite X einer Wulstabschnitt-Befestigungsnut (12a, 12b), die in dem Ultraschallendoskop- (2) - Einführungsabschnitt (3) bereitgestellt ist, um den Ballon (20) zu fixieren, 1,1 <_ W/X <_ 2,5 beträgt, und dass
ein Verhältnis zwischen einem Innendurchmesser Y einer Öffnung des Ballons (20), wobei die Öffnung von dem Wulstabschnitt (21a, 21b) umgeben ist, und einem Außendurchmesser Z eines Teils der Wulstabschnitt-Befestigungsnut (12a, 12b) in dem Ultraschallendoskop- (2) -Einführungsabschnitt (3), wobei der Teil von einer Oberseite einer Wandfläche der Wulstabschnitt-Befestigungsnut (12a, 12b) umgeben ist, 0,30 ≤ Y/Z ≤ 0,55 beträgt.

2. Ultraschallendoskop (2) nach Anspruch 1, wobei das synthetische Elastomer mindestens eines aus einem Elastomer auf Styrolbasis, einem Elastomer auf Olefinbasis, einem Elastomer auf Vinylchloridbasis, einem Elastomer auf Polyesterbasis, einem Elastomer auf Polyurethanbasis, einem Elastomer auf Polyamidbasis, einem Elastomer auf Silikonbasis oder einem Elastomer auf Fluorbasis ist.

3. Ultraschallendoskop (2) nach Anspruch 1 oder 2, wobei das synthetische Elastomer mindestens ein Elastomer auf Olefinbasis einschließt.

4. Ultraschallendoskop (2) nach einem der Ansprüche 1 bis 3, wobei das synthetische Elastomer Isoprenkautschuk einschließt.

5. Ultraschallendoskop (2) nach einem der Ansprüche 1 bis 4, wobei der Ballon (20) zumindest auf einer seiner Außenflächen eine Beschichtung aufweist.

6. Ultraschallendoskop (2) nach Anspruch 5, wobei die Beschichtung aus einem synthetischen Elastomer gebildet ist, das sich von dem synthetischen Elastomer, das den Ballon (20) bildet, unterscheidet.

7. Ultraschallendoskop (2) nach Anspruch 6, wobei das synthetische Elastomer, das zum Bilden der Beschichtung verwendet wird, mindestens eines aus einem Elastomer auf Styrolbasis, einem Elastomer auf Olefinbasis, einem Elastomer auf Vinylchloridbasis, einem Elastomer auf Polyesterbasis, einem Elastomer auf Polyurethanbasis, einem Elastomer auf Polyamidbasis, einem Elastomer auf Silikonbasis oder einem Elastomer auf Fluorbasis ist.

8. Ultraschallendoskop (2) nach Anspruch 6 oder 7, wobei das synthetische Elastomer, das zum Bilden der Beschichtung verwendet wird, mindestens ein Elastomer auf Polyurethanbasis, ein Elastomer auf Silikonbasis oder ein Elastomer auf Fluorbasis ist.

9. Verfahren zur Herstellung eines Ultraschallendoskops (2) mit einem Ballon (20), der so angebracht ist, dass er einen Ultraschall-Sende-Empfangsabschnitt eines Endoskop- (2) -Einführungsabschnitts (3) abdeckt, wobei das Verfahren folgendes umfasst:
Bereitstellen eines Ballons (20) und eines Ultraschallendoskops (2), die die folgenden Beziehungen (a) und (b) erfüllen; und
Anbringen des Ballons (20) an einem Einführungsabschnitt (3) des Ultraschallendoskops (2):
(a) ein Verhältnis zwischen einem Außendurchmesser W eines Wulstabschnitts (21a, 21b) des Ballons (20) und einer Breite X einer Wulstabschnitt-Befestigungsnut (12a, 12b), die in dem Einführungsabschnitt (3) des Ultraschallendoskops (2) bereitgestellt ist, um den Ballon (20) zu fixieren, 1,1 ≤ W/X ≤ 2,5 beträgt; und
(b) ein Verhältnis zwischen einem Innendurchmesser Y einer Öffnung des Ballons (20), wobei die Öffnung von dem Wulstabschnitt (21a, 21b) umgeben ist, und einem Außendurchmesser Z eines Teils der Wulstabschnitt-Befestigungsnut (12a, 12b) in dem Einführungsabschnitt (3) des Ultraschallendoskops (2), wobei der Teil von einer Oberseite einer Wandfläche der Wulstabschnitt-Befestigungsnut (12a, 12b) umgeben ist, 0,30 ≤ Y/Z ≤ 0,55. beträgt.

## Revendications

1. Endoscope (2) à ultrasons comprenant un ballonnet (20), le ballonnet (20) comprenant un élastomère synthétique,
**caractérisé en ce qu'**une relation entre une largeur W d'une partie (21a, 21b) bille du ballonnet (20) et une largeur X d'une rainure (12a, 12b) de fixation de partie bille ménagée dans la section (3) d'insertion de l'endoscope (2) à ultrasons pour fixer le ballonnet (20) est 1,1 ≤ W/X ≤ 2,5, et **en ce que**
une relation entre un diamètre interne Y d'une ouverture du ballonnet (20), l'ouverture étant entourée de la partie (21a, 21b) bille, et un diamètre externe Z d'une partie de la rainure (12a, 12b) de fixation de partie bille dans la section (3) d'insertion de l'endoscope (2) à ultrasons, la partie étant entourée d'un dessus d'une surface de paroi de la rainure (12a, 12b) de fixation de partie bille, est 0,30 ≤ Y/Z ≤ 0,55.

2. Endoscope (2) à ultrasons selon la revendication 1, dans lequel l'élastomère synthétique est au moins l'un d'un élastomère à base de styrène, d'un élastomère à base d'oléfine, d'un élastomère à base de chlorure de vinyle, d'un élastomère à base de polyester, d'un élastomère à base de polyuréthane, d'un élastomère à base de polyamide, d'un élastomère à base de silicone ou d'un élastomère à base de fluor.

3. Endoscope (2) à ultrasons selon la revendication 1 ou la revendication 2, dans lequel l'élastomère synthétique inclut au moins un élastomère à base d'oléfine.

4. Endoscope (2) à ultrasons selon l'une quelconque des revendications 1 à 3, dans lequel l'élastomère synthétique inclut un caoutchouc isoprène.

5. Endoscope (2) à ultrasons selon l'une quelconque des revendications 1 à 4, dans lequel le ballonnet (20) a une couche de revêtement sur au moins une surface externe de celui-ci.

6. Endoscope (2) à ultrasons selon la revendication 5, dans lequel la couche de revêtement est formée d'un élastomère synthétique qui est différent de l'élastomère synthétique formant le ballonnet (20).

7. Endoscope (2) à ultrasons selon la revendication 6, dans lequel l'élastomère synthétique utilisé pour former la couche de revêtement est au moins l'un d'un élastomère à base de styrène, d'un élastomère à base d'oléfine, d'un élastomère à base de chlorure de vinyle, d'un élastomère à base de polyester, d'un élastomère à base de polyuréthane, d'un élastomère à base de polyamide, d'un élastomère à base de silicone ou d'un élastomère à base de fluor.

8. Endoscope (2) à ultrasons selon la revendication 6 ou la revendication 7, dans lequel l'élastomère synthétique utilisé pour former la couche de revêtement est au moins l'un d'un élastomère à base de polyuréthane, d'un élastomère à base de silicone ou d'un élastomère à base de fluor.

9. Procédé de production d'un endoscope (2) à ultrasons avec un ballonnet (20) fixé pour recouvrir une section de transmission-réception d'ultrasons d'une section (3) d'insertion de l'endoscope (2), le procédé comprenant :
la fourniture d'un ballonnet (20) et d'un endoscope (2) à ultrasons qui satisfont les relations (a) et (b) ci-dessous ; et
la fixation du ballonnet (20) à une section (3) d'insertion de l'endoscope (2) à ultrasons :
(a) une relation entre un diamètre externe W d'une partie (21a, 21b) bille du ballonnet (20) et une largeur X d'une rainure (12a, 12b) de fixation de partie bille ménagée dans la section (3) d'insertion de l'endoscope (2) à ultrasons pour fixer le ballonnet (20) est 1,1 ≤ W/X ≤ 2,5 ; et
(b) une relation entre un diamètre interne Y d'une ouverture du ballonnet (20), l'ouverture étant entourée de la partie (21a, 21b) bille, et un diamètre externe Z d'une partie de la rainure (12a, 12b) de fixation de partie bille dans la section (3) d'insertion de l'endoscope (2) à ultrasons, la partie étant entourée d'un dessus d'une surface de paroi de la rainure (12a, 12b) de fixation de partie bille, est 0,30 ≤ Y/Z ≤ 0,55.
